# EUROPEAN PATENT SPECIFICATION

(11) **EP 4 179 925 B1**
(45) Date of publication and mention of the grant of the patent: **27.03.2024**
(21) Application number: 22207051.8
(22) Date of filing: 11.11.2022
(51) Int. Cl.: A47C 1/032, A61G 15/10, A61G 15/02, A61B 90/60

(54) **SEAT, IN PARTICULAR MEDICAL CHAIR**
SITZ, INSBESONDERE MEDIZINISCHER STUHL
SIÈGE, EN PARTICULIER CHAISE MÉDICALE

(30) Priority: 11.11.2021 IT 202100028700
(43) Date of publication of application: 17.05.2023
(73) Proprietor: Tecnodent S.r.l., 40033 Casalecchio di Reno (BO) (IT)
(72) Inventor: TABACCHI, Massimiliano, 35139 PADOVA (IT)
(74) Representative: Susanetto, Carlo

(56) References cited:
- EP-A2- 1 068 838
- EP-A2- 1 161 902
- US-A- 6 019 429

## Description

### Technical field

The present invention relates to a seat, in particular a stool or chair for a healthcare worker or assistant, such as, for example, a medical stool or chair. More specifically, the present invention relates to a dentist stool or chair.

### Technological background

The invention may be applied, in particular, though not exclusively, to the technical field pertaining to seats for professional use, specifically stools or chairs used in the medical environment such as, for example, the so-called dental stools.

Generally, stools offer the possibility of adjusting the inclination of the seat member and/or backrest, which allows the user of the stool or chair to achieve a correct positioning on the seat member. The possibility for the user to operate without obstacles deriving from the seat is another aspect that is highly appreciated in the working environment. Ergonomics therefore plays a key role when choosing a seat for professional use.

The Applicant observed, however, that the current adjustable seats suffer from certain limitations, such as comfort and hygiene when adjusting the seat member and/or backrest inclination, or the excessive time required for the operator to make such adjustments, due to the current adjustment systems they are provided with.

Prior art examples related to the invention are disclosed in EP1068838A2, US6019429A and EP1161902A2.

The problem underlying the present invention is that of making available a seat that is structurally and functionally conceived to at least partially obviate one or more of the drawbacks referred to with reference to the mentioned prior art.

In the context of this problem, an object of the present invention is to provide a seat that allows to adjust in a quicker and/or easier and/or more hygienic way the inclination of the seat member and/or backrest.

This problem is solved and this object is achieved by the invention by means of a seat according to one or more of the accompanying claims.

### Summary of the invention

The present invention, in a first aspect thereof, refers to a seat, in particular to a stool or chair for a healthcare worker or assistant such as, for example, a medical stool or chair, more particularly to a dentist stool or chair. According to an embodiment of the invention, the seat comprises:
- a seat member which is secured to a base with the ability to rotate with respect to said base about a first rotation axis,
- a backrest which is secured to said seat member with the ability to rotate with respect to said seat member about a second rotation axis, the backrest being provided with a support surface which extends from a lower edge thereof to an upper edge thereof opposite the lower edge, said support surface delimiting with said seat member a receiving zone apt to receive a user of said seat,
- a reclining mechanism comprising:
   - at least one control member which is arranged laterally to said backrest, preferably beside the latter with respect to said support surface, more preferably when said seat is in use, wherein said at least one control member is movable with respect to said backrest between a first position and a second position, the first position being closer to said receiving zone than the second position, and
   - at least one actuator which is operatively connected to one of said seat member and said backrest and which is provided to urge said one of said seat member and said backrest in terms of rotation about the respective rotation axis,
wherein said at least one control member is operatively connected to said at least one actuator and is provided to actuate said at least one actuator in order to urge said one of said seat member and said backrest in terms of rotation about the respective rotation axis when said at least one control member is in the second position, wherein at least one control member is secured to said backrest.

In particular, said at least one control member is secured to said backrest without interposing said at least one actuator,

The seat may also have at least one of the preferred characteristics set forth hereinafter. In particular, the seat may comprise any combination of two or more of the preferred characteristics set forth hereinafter.

Preferably, said at least one control member is secured to said backrest in such a way that the rotation of said backrest about said second rotation axis causes a displacement, more preferably a rotation, of the at least one control member about said second rotation axis.

Preferably, said at least one control member includes an operating surface. Preferably, said operating surface is directed towards said receiving zone when said at least one control member is in said first position.

Preferably, said operating surface of said at least one control member delimits, with said support surface and said seat member, said receiving zone when said at least one control member is in said first position.

In other words, the receiving zone preferably extends to the area occupied by said operating surface when said at least one control member is in said first position.

Preferably, said operating surface is intended to be moved directly or indirectly, more preferably directly, by a user received in that receiving zone. Preferably, said operating surface is arranged in a position above said seat member in relation to said base, more preferably when said seat is in use. Preferably, said operating surface is oriented towards a reference plane opposite to said support surface with respect to said seat member, more preferably when said seat is in use.

Preferably, said reference plane is a vertical plane.

Preferably, said operating surface is substantially parallel to said support surface or is inclined with respect to said support surface by an angle lower than or equal to 40°, more preferably lower than or equal to 30°, more preferably lower than or equal to 20°, more preferably lower than or equal to 10°.

Preferably, said operating surface comprises a contact portion which is spaced apart from an upper surface of said seat member by at least 19 cm and/or at a maximum by 32 cm when said at least one control member is in the first position, and wherein the contact portion is spaced apart from a reference axis of said backrest by at least 23 cm and/or at a maximum by 32 cm when said at least one control member is in the first position, said reference axis passing through the lower edge and the upper edge of said support surface centrally with respect to the upper surface.

Preferably, said operating surface is located behind said support surface of said backrest with respect to said receiving zone when said at least one control member is in said first position and/or in said second position.

Preferably, said seat includes an elastic return device operatively connected to said at least one control member and provided to move said at least one control member from said second position to said first position when external pressures do not act on said operating surface.

Preferably, said reclining mechanism comprises a frame which is provided with said at least one control member and which is secured to said backrest with the ability to rotate with respect to said backrest about a third rotation axis in such a manner that said at least one control member rotates towards a zone behind said seat and opposite the receiving zone with respect to said backrest when said at least one control member is moved from said first position to said second position.

Preferably, said at least one actuator comprises a gas spring which is hinged on said seat member and said base in order to rotate said seat member about said first rotation axis and said at least one control member is provided to actuate said gas spring in order to urge said seat member in terms of rotation about said first rotation axis when said at least one control member is in the second position.

Preferably, said at least one actuator comprises a gas spring which is hinged on said seat member and said backrest in order to rotate said backrest about said second rotation axis and said at least one control member is provided to actuate said gas spring in order to urge said backrest in terms of rotation about said second rotation axis when said at least one control member is in the second position.

Preferably, said reclining mechanism comprises a cable device associated with said at least one control member and said at least one actuator in order to actuate the respective gas spring when said at least one control member is in the second position, said cable device preferably comprising a Bowden cable. Preferably, said at least one actuator comprises a first actuator operatively connected to said seat member and a second actuator operatively connected to said backrest, and said at least one control member comprises a first control member and a second control member arranged opposite to said first control member with respect to said backrest, wherein said first control member and said second control member are operatively connected to said first actuator and said second actuator, respectively, to urge said seat member and said backrest respectively, in terms of rotation about their respective rotation axes when said first control member and said second control member respectively are in said second position.

Preferably, said frame of said reclining mechanism is T-shaped and comprises a central member from which two frame portions which are provided with said first control member and said second control member, respectively, extend in opposite directions, said central member of said frame being secured to said backrest with the ability to rotate about said third rotation axis.

Preferably, said first control member and said second control member comprise respective operating surfaces, each operating surface comprising a first lateral end which is proximal with respect to said backrest and a second lateral end which is distal with respect to said backrest and opposite the first lateral end, wherein the distance between said first lateral end of the first control member and said first lateral end of the second control member is at a maximum 40 cm and/or the distance between said second lateral end of the first control member and said second lateral end of the second control member is at least 50 cm.

Preferred characteristics of the invention are defined in the dependent claims.

### Brief description of the drawings

Characteristics and further advantages of the invention will be clearer from the following detailed description of its preferred, though non-exclusive, embodiments, which are shown, for exemplary and non-limiting purposes, with reference to the accompanying drawings wherein:
- Figure 1 is a schematic representation of a seat according to an embodiment of the invention,
- Figures 2 and 3 are views from above of the seat of Figure 1 in two different operating conditions,
- Figures 4 and 5 show details of the seat of Figure 1,
- Figures 6, 7 and 8 are partial perspective, side and front views of the seat of Figure 1,
- Figure 9 is a view of a detail of a seat according to an embodiment of the invention, and
- Figure 10 is a cross-section of a seat component according to an embodiment of the invention.

### Description of embodiments of the invention

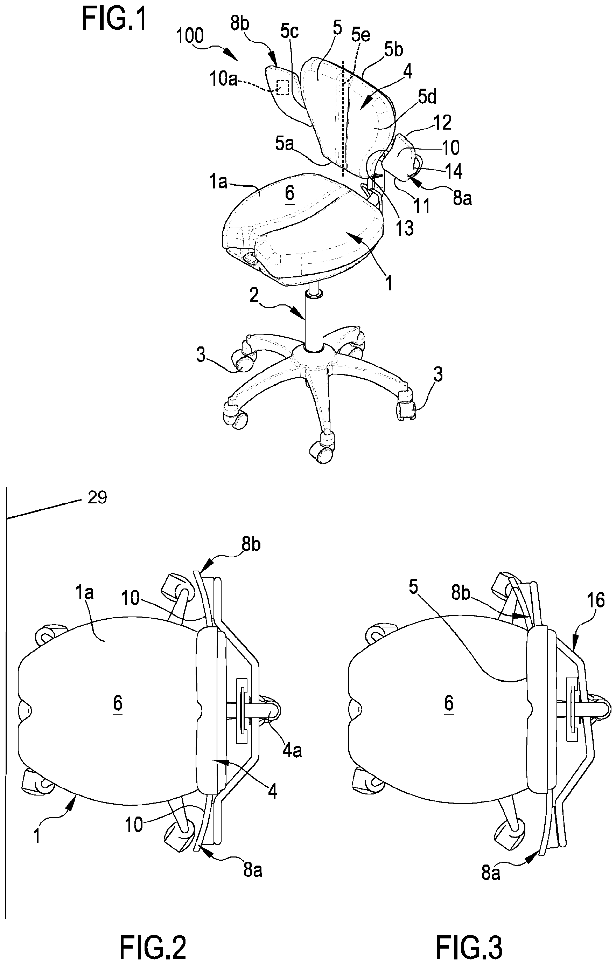

Referring initially to Figure 1, number 100 globally indicates a seat according to an embodiment of the invention.

According to an aspect of the invention, the seat 100 comprises a seat member 1 secured to a base 2 with the ability to rotate with respect to the base 2 about a first rotation axis X.

In particular, the first rotation axis X is substantially horizontal when the seat 100 is in use.

Preferably, the seat 100 is a health worker's or assistant's stool or chair, more preferably a medical stool or chair, in particular a dental stool or chair.

The seat 100 may be considered to be in use when it is placed on a support surface by means of the base 2. Preferably, the support surface is substantially horizontal.

The seat 100 is intended to rest on the support surface by means of the base 2, which is preferably provided with castors 3 that allow easy movement of the chair 100 on the flat surface.

The base 2 keeps the seat member 1 raised from the support surface when the seat 100 is in use.

Preferably, the base 2 includes a telescopic portion 2a to adjust the height of the seat member 1 from the flat surface on which the seat 100 rests when in use.

The seat 100 also includes a backrest 4 that is secured to the seat member 1 with the ability to rotate with respect to the seat member 1 around a second rotation axis Y.

In particular, the second rotation axis Y is substantially horizontal.

The backrest 4 is provided with a support surface 5 extending from a lower edge 5a thereof to an upper edge 5b thereof opposite the lower edge 5b. The lower edge 5a is lower, i.e. closer to the support surface, than the upper edge 5b when the seat 100 is in use.

The support surface 5 is delimited laterally by a first lateral edge 5c and a second lateral edge 5d, opposite to the first lateral edge 5c.

The first lateral edge 5c and the second lateral edge 5d extend from the lower edge 5a to the upper edge 5b of the support surface 5.

The support surface 5 delimits with the seat member 1, in particular with an upper surface 1a of the seat member 1, a receiving zone 6 apt to receive a user of the seat 100.

The upper surface 1a is the surface of the seat member 1 oriented upwards when the seat 100 is in use.

According to an embodiment of the invention, the seat 100 comprises a reclining mechanism 7.

The reclining mechanism 7 includes at least one control member 8a, 8b arranged at the side of the backrest 4.

In other words, the at least one control member 8a, 8b is arranged beside the backrest 4 with respect to the support surface 5 when the seat 100 is in use. The at least one control member 8a, 8b is movable relative to the backrest 4 between a first position and a second position, the first position being closer to the receiving zone 6 than the second position.

Figures 2 and 3 show a control member 8a in the first position and in the second position, respectively, according to an embodiment of the invention.

Preferably, the at least one control member 8a, 8b is secured to the backrest 4. Thus, a displacement of the backrest 4 with respect to the base 2 and/or seat member 1 causes a displacement of the at least one control member 8a, 8b with respect to the base 2 and/or seat member 1.

Preferably, the at least one control member 8a, 8b is secured to the backrest 4 in such a way that the rotation of the backrest 4 about the second rotation axis Y causes a displacement (more preferably a rotation) of the at least one control member 8a, 8b about the second rotation axis Y.

The reclining mechanism 7 further comprises at least one actuator 9a, 9b operatively connected to one of the seat member 1 and the backrest 4 and provided to urge the one of the seat member 1 and the backrest 4 in terms of rotation about the respective rotation axis of rotation, preferably, along a predetermined rotation direction.

According to an aspect of the invention, the at least one control member 8a, 8b is operatively connected to the at least one actuator 9a, 9b and is provided to actuate the at least one actuator 9a, 9b in order to urge the one of the seat member 1 and the backrest 4 in terms of rotation about the respective rotation axis when the at least one control member 8a, 8b is in the second position. Therefore, the at least one actuator 9a, 9b acts on the one of the seat member 1 and the backrest 4, leading it to rotate about the respective rotation axis and, preferably, along a predetermined rotation direction when the at least one actuator 9a, 9b is actuated by the at least one control device 8a, 8b. The seat member 1 or backrest 4 may be rotated either along a predetermined rotation direction through the (direct) action of at least one actuator 9a, 9b on the seat member 1 or backrest 4, or along a second rotation direction opposite to the predetermined one.

The rotation of the one of the seat member 1 and the backrest 4 along the second rotation direction may be obtained by moving the at least one control member 8a, 8b from the first position to the second position and acting on the seat 100 in such a way as to impart a force on the seat member 1 and/or the backrest 4 such as to counteract and overcome the action exerted by the at least one actuator 9a, 9b on the one of the seat member 1 and the backrest 4.

It should be noted that the characteristics of the seat 100, in particular the arrangement of the at least one control member 8a, 8b at the side of the backrest 4, allow the user thereof to act on the control member with an elbow rather than using the hands and, therefore, to quickly and easily adjust the inclination of the seat member or backrest.

Furthermore, using the elbow in order to adjust the inclination of the seat member 1 or backrest 4 makes the reclining mechanism 7 a more hygienic solution than known adjustment systems.

According to an embodiment of the invention, the at least one actuator 9a, 9b is provided to stop the rotation of the one of the seat member 1 and the backrest 4 about the respective rotation axis when the at least one control member 8a, 8b is in the first position.

According to an embodiment of the invention, the at least one control member 8a, 8b comprises an operating surface 10 which is directed towards the receiving zone 6 when the at least one control member 8a, 8b is in the first position.

The operating zone 10 is intended to be moved directly or indirectly by a user received in receiving zone 6.

The user received in the receiving zone 6is thereby eased to operate the reclining mechanism 7.

Specifically, the user, once positioned in the receiving zone 6, may exert a pressure on the operating surface 10 with his/her elbow such as to move the relevant control member from the first position to the second position, thereby operating the actuator without using his/her hands.

The operating surface 10 is arranged in a position above the seat member 1 in relation to the base 2, as clearly shown in Figures 1-3. It is also clear from

Figures 1-3, that the operating surface 10 is directed towards a reference plane 29 opposite the support surface 5 with respect to the seat member 1.

According to an embodiment of the invention, the operating surface 10 comprises a contact portion 10a.

Preferably, the contact portion 10a is spaced apart from the upper surface 1a of the seat member 1 by at least 19 cm and/or at a maximum by 32 cm, more preferably the contact portion 10a is spaced apart from the upper surface 1a by 25 cm, when the at least one control member 8a, 8b is in the first position. Preferably, the contact portion 10a is spaced apart from a reference axis 5e of the backrest 4 by at least 23 cm and/or at a maximum by 32 cm when the at least one control member (8a, 8b) is in the first position.

The reference axis 5e passes through the lower edge 5a and the upper edge 5b of the support surface 5 centrally with respect to the upper surface 1a. Such characteristics allow to obtain an operating surface 10 that is sufficiently large and arranged so as to allow the user of the seat 100 to operate the reclining mechanism 7 particularly easily using his/her elbow.

Preferably, the term "distance" between the contact portion 10a and the upper surface 1a and between the contact portion 10a and the reference axis 5e refers to the minimum distance between such elements.

Preferably, the reference axis 5e is equally spaced apart from the first lateral edge 5c and the second lateral edge 5d of the support surface 5.

Preferably, the reference axis 5e corresponds to an axis of symmetry of the support surface 5.

Preferably, the reference axis 5e extends at the spinal column of a user of seat 100 when this is received in the receiving zone 6.

Preferably, the reference axis 5e is transverse, more preferably perpendicular, to the upper surface 1a of the seat member 1.

According to an embodiment of the invention, shown by way of example in the Figures 1-3, the operating surface 10 comprises a lower end 11 and an upper end 12 opposite to the lower end.

The lower end 11 is intended to be directed downwards, i.e. towards the support surface on which the seat 100 rests when it is in use.

According to an embodiment of the invention, the operating surface 10 comprises a first lateral end 13 proximal to the backrest 4 and a second lateral end 14 distal to the backrest 4 and opposite the first lateral end 13. Preferably, the first lateral end 13 and the second lateral end 14 extend from the lower surface 11 to the upper surface 12.

Preferably, the ends 11, 12, 13 and 14 of the operating surface 10 determine the contour of the at least one control member 8a, 8b seen from the receiving zone 6.

Preferably, the lower end 11 is spaced apart from the seat member 1 at a maximum by 19 cm and/or the upper end 12 is spaced apart from the seat member by at least 32 cm.

Preferably, the first lateral end 13 is spaced apart from the backrest 4 at a maximum by 2 cm and/or the second lateral end 14 is spaced apart from the backrest 4 by at least 11 cm when the at least one control member 8a, 8b is in the first position.

Such characteristics allow to obtain a particularly ergonomic operating surface 10 that allows the user of the seat 100 to operate the reclining mechanism 7 particularly easily using his/her elbow.

According to an embodiment of the invention, the operating surface 10 is located behind the support surface 5 of the backrest 4 with respect to the receiving zone 6 when the at least one control member 8a, 8b is in the first position and/or in the second position.

This is shown by way of example in Figure 3, in relation to the control member 8a.

This characteristic is particularly useful so that the operating surface 10 does not hinder the user of the seat 100 when placed in receiving zone 6.

According to an embodiment of the invention, the reclining mechanism 7 comprises a frame 16 provided with the at least one control member 8a, 8b and secured to the backrest 4 with the ability to rotate relative to the backrest 4 about a third rotation axis Z such that the at least one control member 8a, 8b rotates towards a zone behind the seat 100 and opposite the receiving zone 6 relative to the backrest 4 when the at least one control member 8a, 8b is moved from the first position to the second position. Preferably, the third rotation axis Z extends transverse to the upper surface 1a of the seat member 1.

The frame 16 secured to the backrest 4 allows the at least one control member 8a, 8b to be rotationally integral to the backrest 4 with respect to the second rotation axis Y.

Thus, the orientation and spatial position of the at least one control member 8a, 8b with respect to the backrest 4 remains constant as the inclination of the latter with respect to the seat member 1 changes.

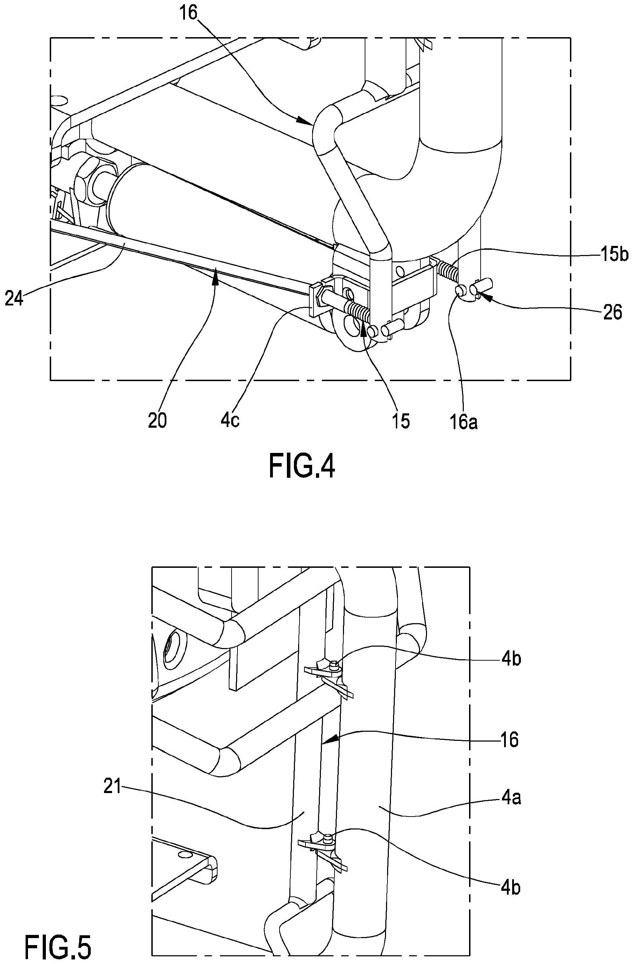

With reference to Figure 5, the frame 16 may be hinged to the backrest 4. Preferably, the frame 16 is connected to a framework 4a of the backrest 4 by one or more pins 4b.

According to an embodiment of the invention, the seat 100 comprises an elastic return device 15, in particular a coil spring, operatively connected to the at least one control member 8a, 8b and provided to move the at least one control member 8a, 8b from the second position to the first position when external pressures (i.e. pressures exerted by the user of the seat 100 on the operating surface 10) do not act on the operating surface 10.

This characteristic allows the control member to remain in the first position, making it easier for the user of the seat 100 to operate the reclining mechanism 7 when necessary.

Preferably, the elastic return device 15 is arranged in such a way as to take on a rest condition when the at least one control member 8a, 8b is in the first position and an operating condition when the at least one control member 8a, 8b is in the second position. In the rest condition the elastic return device 15 takes on a balanced configuration while in the operating condition the elastic return device 15 takes on an elongated or compressed configuration. Therefore, if no external pressures are present on the operating surface 10, the elastic return device 15 will tend to the rest condition by moving and/or maintaining the at least one control member 8a, 8b in the first position.

Figure 4 shows an example of an elastic return device 15.

The elastic return device 15 may be placed between a matching portion 16a of the frame 16 and the framework 4a of the backrest 4.

In particular, the matching portion 16a may be identified in a threaded pin housed in a location of the frame 16.

In particular, the elastic return device 15 may be arranged between the matching portion 16a of the frame 16 and a bracket 4c of the framework 4a. Preferably, the elastic return device 15 (e.g. a coil spring) is arranged laterally to the rotation axis Z, more preferably on the opposite side of the control member 8a, 8b associated to that axis.

Preferably, the matching portion 16a extends in such a way that it is brought closer to the framework 4a when the at least one control member 8a, 8b is moved from the first position to the second position, thereby compressing the elastic return device 15.

When no external pressures act on the operating surface 10, the elastic return device 15 returns to its rest condition by urging the matching portion 16a in terms of moving away from the framework 4a and thus causing the at least one control member 8a, 8b to move from the second position to the first position.

According to a preferred embodiment of the invention, the at least one actuator comprises a first actuator 9a operatively connected to the seat member 1 and a second actuator 9b operatively connected to the backrest 4. The at least one control member comprises a first control member 8a and a second control member 8b arranged opposite the first control member 8a with respect to the backrest 4.

The first control member 8a and the second control member 8b are operatively connected to the first actuator 9a and the second actuator 8b, respectively, in order to urge the seat member 1 and the backrest 4 respectively, in terms of rotation about their respective rotation axes X and Y when the first control member 8a and the second control member 8b, respectively, are in the second position.

Figures 1-3 show a preferred embodiment of the seat 100 comprising a pair of control members 8a, 8b and a pair of actuators 9a, 9b.

Preferably, the first control member 8a and the second control member 8b comprise respective operating surfaces 10.

Preferably, the distance between the first lateral end 13 of the operating surface 10 of the first control member 8a and the first lateral end 13 of the operating surface 10 of the second control member 8b is at a maximum 40 cm, more preferably at a maximum 48 cm.

Preferably, the distance between the second lateral end 14 of the operating surface 10 of the first control member 8a and the second lateral end 14 of the operating surface 10 of the second control member 8b is at least 50 cm, more preferably at least 58 cm.

Preferably, the first control member 8a and the second control member 8b comprise respective contact portions 10a.

Preferably, the term "distance" between the first lateral ends 13 of the control members 8a, 8b and between the second lateral ends 14 of the control members 8a, 8b refers to the minimum distance between such elements. According to an embodiment of the invention, the frame 16 of the reclining mechanism 7 is T-shaped and comprises a central member 21 from which two frame portions 22, 23 provided with the first control member 8a and the second control member 8b, respectively, extend in opposite directions. The central member 21 of the frame 16 preferably extends along the third rotation axis and is secured to the backrest 4 with the ability to rotate around the third rotation axis Z.

This allows the reclining mechanism 7 to be integral with the backrest 4 when reclining the latter.

Furthermore, the provision of the T-shaped frame allows to obtain a single-piece structure, which is a structurally simpler solution with a reduced number of components compared to a solution involving two separate and independent portions associated with respective control members.

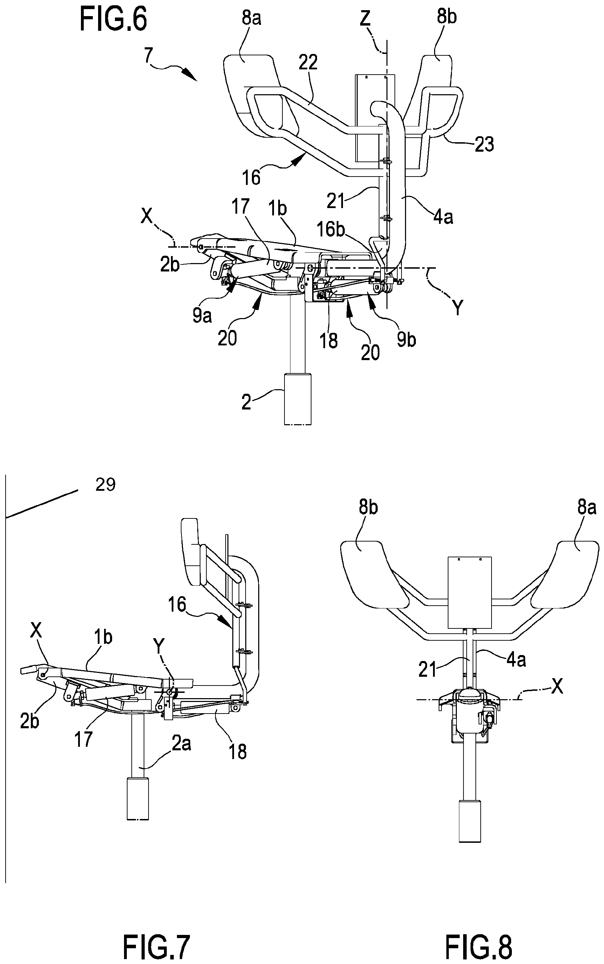

Figures 6-8 show an embodiment of the frame 16 provided with the first and second control members 8a, 8b.

In this case, the central member 21 of the frame 16 is preferably connected to the framework 4a of the backrest 4 by one or more pins 4b.

Preferably, the frame 16 comprises an end portion provided with a through-hole 16b and extended from the central member 21 towards the base 2. The framework 4a extends through the through-hole 16b so that the matching portion 16a of the frame 16 is arranged on the side opposite the central member 21 with respect to the framework 4a.

In addition or alternatively, the elastic return device 15 preferably comprises a pair of springs 15a, 15b arranged on opposite sides with respect to the central member 21 of the frame 16.

Thereby, a first spring 15a of the pair of springs is compressed by the matching portion 16a of the frame 16 when the first control member 8a is moved from the first position to the second position while the second spring 15b of the pair of springs is compressed when the second control member 8b is moved from the first position to the second position.

When no external pressures act on the operating surface 10 of the first control member 8a, the first spring 15a returns to its rest condition, causing the first control member 8a to move from the second position to the first position. Similarly, the second spring 15b returns to its rest condition, determining the movement of the second control member 8b from the second position to the first position when external pressures on the operating surface 10 of the second control member 8b are removed.

According to an embodiment of the invention, the at least one actuator, preferably the first actuator 9a, comprises a gas spring 17 hinged to the seat member 1 (in particular to a frame 1b of the seat member 1) and to the base 2 (in particular to a frame 2b of the base 2) to rotate the seat member 1 about the first rotation axis X. The at least one control member, preferably the first control member 8a, is provided to actuate the gas spring 17 to urge the seat member 1 in terms of rotation about the first rotation axis X when the at least one control member 8a is in the second position.

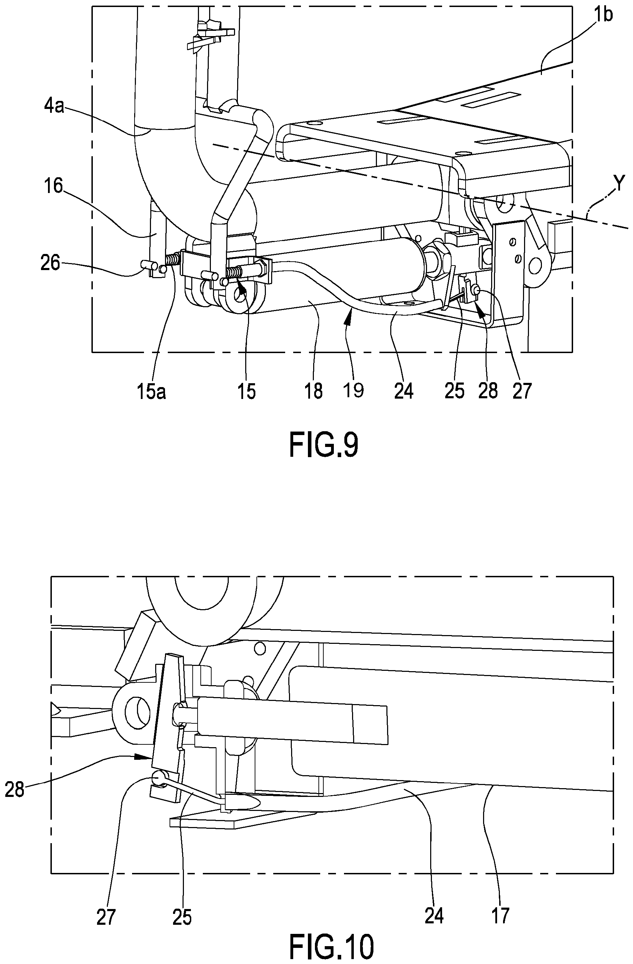

According to an embodiment of the invention, the at least an actuator, preferably the second actuator 9b, comprises a gas spring 18 hinged to the seat member 1 (in particular to the frame 1b of the seat member1) and to the backrest 4 (in particular to the framework 4a of the backrest 4) to rotate the backrest 4 about the second rotation axis Y. The at least a control member, preferably the second control member 8b, is provided to actuate the gas spring 18 to urge the backrest 4 in terms of rotation about the second rotation axis Y when the at least one control member 8b is in the second position. Figures 6-10 show details of the seat 100 provided with the first and second actuators 9a, 9b according to an embodiment of the invention.

According to an embodiment of the invention, the reclining mechanism 7 of the seat 100 comprises a cable device 19 associated with the at least one control member 8a, 8b and the at least one actuator 9a, 9b for actuating the respective gas spring 17, 18 when the at least one control member 8a, 8b is in the second position. Preferably, the cable device 19 comprises a Bowden cable 20.

It should be noted that the rotation of the backrest in relation to the seat member may lead to a change in the distance and/or position between the control member and the actuator.

Thanks to its flexible characteristics, the cable device 19 may be easily adapted to different configurations of the seat 100 by rotating the backrest relative to the seat.

The Bowden cable 20 comprises a sheath 24 and a cable 25 sliding inside the sheath.

Referring in particular to Figure 4, a first longitudinal end (head) 26 of the cable 25 is fixed to the frame 16, in particular at a zone of the matching portion 16a extended on the same side, with respect to the rotation axis Z, of the control member 8a, 8b associated with the cable device 19, while a second longitudinal end 27 of the cable 25 opposite the first end 26 is fixed to a trigger 28 arranged to actuate the gas spring of the at least one actuator 9a, 9b. Therefore, the cable 22 is pulled by the frame 16 while the at least one control member 8a, 8b moves from the first position to the second position, thus rotating and activating the trigger 25 connected thereto with the consequent opening and actuation of the gas spring.

In case the seat 100 comprises a first and second control member 8a, 8b and a first and second actuator 9a, 9b, the cable device 19 preferably comprises a first and second Bowden cable 20 connected to the triggers 25 associated with the gas springs 17, 18 of the respective actuators 9a, 9b.

The invention thus solves the problem set forth while achieving a plurality of the above-mentioned advantages.

## Claims

1. Seat (100) comprising:
• a seat member (1) which is secured to a base (2) with the ability to rotate with respect to said base (2) about a first rotation axis (X),
• a backrest (4) which is secured to said seat member (1) with the ability to rotate with respect to said seat member (1) about a second rotation axis (Y), the backrest (4) being provided with a support surface (5) which extends from a lower edge (5a) thereof to an upper edge (5b) thereof opposite said lower edge (5a), said support surface (5) delimiting with said seat member (1) a receiving zone (6) apt to receive a user of said seat (100),
• a reclining mechanism (7) comprising:
• at least one control member (8a; 8b) which is arranged laterally to said backrest (4), beside the latter with respect to said support surface (5), wherein said at least one control member (8a; 8b) is movable with respect to the backrest (4) between a first position and a second position, the first position being closer to said receiving zone (6) than the second position, and
• at least one actuator (9a; 9b) which is operatively connected to one of said seat member (1) and said backrest (4) and which is provided to urge the one of said seat member (1) and said backrest (4) in terms of rotation about the respective rotation axis (X,Y),
wherein said at least one control member (8a; 8b) is operatively connected to said at least one actuator (9a; 9b) and is provided to actuate said at least one actuator (9a; 9b) in order to urge said one of said seat member (1) and said backrest (4) in terms of rotation about the respective rotation axis (X,Y) when said at least one control member (8a; 8b) is in the second position, **characterised in that** said at least one control member (8a; 8b) is secured to said backrest (4).

2. Seat according to claim 1, wherein said at least one control member (8a; 8b) is secured to said backrest (4) such that the rotation of said backrest (4) about said second rotation axis (Y) causes a displacement, preferably a rotation, of the at least one control member (8a; 8b) about said second rotation axis (Y).

3. Seat according to any one of the preceding claims, wherein said at least one control member (8a; 8b) comprises an operating surface (10) directed towards said receiving zone (6) when said at least one control member (8a; 8b) is in said first position.

4. Seat according to claim 3, wherein said operating surface (10) is arranged in a position above said seat member (1) with respect to said base (2).

5. Seat according to claim 3 or 4, wherein said operating surface (10) is directed towards a reference plane (29) opposite said support surface (5) with respect to said seat member (1).

6. Seat according to any one of claims 3 to 5, wherein said operating surface (10) comprises a contact portion (10a) which is spaced apart from an upper surface (1a) of the said member (1) by at least 19 cm and/or at a maximum by 32 cm when said at least one control member (8a, 8b) is in the first position, and wherein said contact portion (10a) is spaced apart from a reference axis (5e) of said backrest (4) by at least 23 cm and/or at a maximum by 32 cm when said at least one control member (8a, 8b) is in the first position, said reference axis (5e) passing through the lower edge (5a) and the upper edge (5b) of said support surface (5) centrally with respect to said upper surface (1a).

7. Seat according to any one of claims 3 to 6, wherein said operating surface (10) is located behind said support surface (5) of said backrest (4) with respect to said receiving zone (6) when said at least one control member (8a; 8b) is in said first position and/or in said second position.

8. Seat according to any one of claims 3 to 7, wherein said seat (100) comprises an elastic return device (15) operatively connected to said at least one control member (8a; 8b) and provided to move said at least one control member (8a; 8b) from said second position to said first position when external pressure do not act on said operating surface (10).

9. Seat according to any one of the preceding claims, wherein said reclining mechanism (7) comprises a frame (16) which is provided with said at least one control member (8a; 8b) and which is secured to said backrest (4) with the ability to rotate with respect to said backrest (4) about a third rotation axis (Z) in such a manner that said at least one control member (8a; 8b) rotates towards a zone behind said seat (100) and opposite the receiving zone (6) with respect to said backrest (4) when said at least one control member (8a; 8b) is moved from said first position to said second position.

10. Seat according to any one of the preceding claims, wherein said at least one actuator (9a) comprises a gas spring (17) which is hinged on said seat member (1) and said base (2) in order to rotate said seat member (1) about said first rotation axis (X) and said at least one control member (8a) is provided to actuate said gas spring (17) in order to urge said seat member (1) in terms of rotation about said first rotation axis (X) when said at least one control member (8a) is in the second position.

11. Seat according to any one of the preceding claims, wherein said at least one actuator (9b) comprises a gas spring (18) which is hinged on said seat member (1) and said backrest (4) in order to rotate said backrest (4) about said second rotation axis (Y) and said at least one control member (8b) is provided to actuate said gas spring (18) in order to urge said backrest (4) in terms of rotation about said second rotation axis (Y) when said at least one control member (8b) is in the second position.

12. Seat according to any one of the preceding claims, wherein said at least one actuator comprises a first actuator (9a) operatively connected to said seat member (1) and a second actuator (9b) operatively connected to said backrest (4), and said at least one control member comprises a first control member (8a) and a second control member (8b) arranged opposite said first control member (8a) with respect to said backrest (4), wherein said first control member (8a) and said second control member (8b) are operatively connected to said first actuator (9a) and, respectively, said second actuator (9b) to urge said seat member (1) and said backrest (4) respectively, in terms of rotation about respective rotation axes when said first control member (8a) and said second control member (8b) respectively are in the second position.

13. Seat according to claim 12 wherein said frame (16) of said reclining mechanism (7) is T-shaped and comprises a central member (21), from which two frame portions (22, 23) which are provided with said first control member (8a) and said second control member (8b), respectively, extend in opposite directions, said central member (21) of said frame (16) being secured to said backrest (4) with the ability to rotate about said third rotation axis (Z).

14. Seat according to claim 12 or 13, wherein said first control member (8a) and said second control member (8b) comprise respective operating surfaces (10), each operating surface (10) comprising a first lateral end (13) which is proximal with respect to said backrest (4) and a second lateral end (14) which is distal with respect to said backrest (4) and opposite said first lateral end (13), wherein the distance between said first lateral end (13) of the first control member (8a) and said first lateral end (13) of the second control member (8b) is at a maximum 40 cm and/or the distance between said second lateral end (14) of the first control member (8a) and said second lateral end (14) of the second control member (8b) is at least 50 cm.

## Patentansprüche

1. Sitz (100), umfassend:
- ein Sitzelement (1), das an einer Basis (2) mit der Möglichkeit befestigt ist, um sich in Bezug auf die Basis (2) um eine erste Drehachse (X) zu drehen,
- eine Rückenlehne (4), die am Sitzelement (1) mit der Möglichkeit befestigt ist, um sich in Bezug auf das Sitzelement (1) um eine zweite Drehachse (Y) zu drehen, wobei die Rückenlehne (4) mit einer Stützfläche versehen ist (5), die sich von einer Unterkante (5a) bis zu einer Oberkante (5b), die sich gegenüber der Unterkante (5a) befindet, erstreckt, wobei die Stützfläche (5) mit dem Sitzelement (1) einen Aufnahmebereich (6) begrenzt, der geeignet ist, um einen Benutzer des Sitzes (100) aufzunehmen,
- einen Verstellmechanismus (7), umfassend:
- zumindest ein Steuerelement (8a; 8b), das seitlich an der Rückenlehne (4) und in Bezug auf die Stützfläche (5) neben dieser angeordnet ist, wobei das zumindest eine Steuerelement (8a; 8b) in Bezug auf die Rückenlehne (4) zwischen einer ersten Position und einer zweiten Position beweglich ist, wobei die erste Position näher am Aufnahmebereich (6) liegt als die zweite Position, und
- zumindest einen Aktuator (9a; 9b), der mit dem Sitzelement (1) oder der Rückenlehne (4) wirkverbunden und vorgesehen ist, um das Sitzelement (1) oder die Rückenlehne (4) in Bezug auf eine Drehung um die jeweilige Drehachse (X, Y) zu bewegen,
wobei das zumindest eine Steuerelement (8a; 8b) mit dem zumindest einen Aktuator (9a; 9b) wirkverbunden und vorgesehen ist, um den zumindest einen Aktuator (9a; 9b) zu betätigen, um das Sitzelement (1) oder die Rückenlehne (4) in Bezug auf eine Drehung um die jeweilige Drehachse (X, Y) zu bewegen,
wenn sich das zumindest eine Steuerelement (8a; 8b) in der zweiten Position befindet, **dadurch gekennzeichnet, dass** das zumindest eine Steuerelement (8a; 8b) an der Rückenlehne (4) befestigt ist.

2. Sitz nach Anspruch 1, wobei das zumindest eine Steuerelement (8a; 8b) an der Rückenlehne (4) befestigt ist, so dass die Drehung der Rückenlehne (4) um die zweite Drehachse (Y) eine Verschiebung, vorzugsweise eine Drehung, des zumindest einen Steuerelements (8a; 8b) um die zweite Drehachse (Y) bewirkt.

3. Sitz nach einem der vorhergehenden Ansprüche, wobei das zumindest eine Steuerelement (8a; 8b) eine Betätigungsfläche (10) aufweist, die auf den Aufnahmebereich (6) gerichtet ist, wenn sich das zumindest eine Steuerelement (8a; 8b) in der ersten Position befindet.

4. Sitz nach Anspruch 3, wobei die Betätigungsfläche (10) in einer Position über dem Sitzelement (1) in Bezug auf die Basis (2) angeordnet ist.

5. Sitz nach Anspruch 3 oder 4, wobei die Betätigungsfläche (10) auf eine Bezugsebene (29), die der Stützfläche (5) gegenüberliegt, in Bezug auf das Sitzelement (1) gerichtet ist.

6. Sitz nach einem der Ansprüche 3 bis 5, wobei die Betätigungsfläche (10) einen Kontaktabschnitt (10a) aufweist, der von einer oberen Fläche (1a) des Elements (1) um zumindest 19 cm und/oder maximal um 32 cm beabstandet ist, wenn sich das zumindest eine Steuerelement (8a, 8b) in der ersten Position befindet, und wobei der Kontaktabschnitt (10a) von einer Referenzachse (5e) der Rückenlehne (4) um zumindest 23 cm und/oder maximal um 32 cm beabstandet ist, wenn sich das zumindest eine Steuerelement (8a, 8b) in der ersten Position befindet, wobei die Referenzachse (5e) durch die Unterkante (5a) und die Oberkante (5b) der Stützfläche (5) mittig in Bezug auf die obere Fläche (1a) verläuft.

7. Sitz nach einem der Ansprüche 3 bis 6, wobei die Betätigungsfläche (10) hinter der Stützfläche (5) der Rückenlehne (4) in Bezug auf den Aufnahmebereich (6) angeordnet ist, wenn sich das zumindest eine Steuerelement (8a; 8b) in der ersten Position und/oder in der zweiten Position befindet.

8. Sitz nach einem der Ansprüche 3 bis 7, wobei der Sitz (100) eine elastische Rückstellvorrichtung (15) aufweist, die mit dem zumindest einen Steuerelement (8a; 8b) wirkverbunden und vorgesehen ist, um das zumindest eine Steuerelement (8a; 8b) von der zweiten Position in die erste Position zu bewegen, wenn kein äußerer Druck auf die Betätigungsfläche (10) wirkt.

9. Sitz nach einem der vorhergehenden Ansprüche, wobei der Neigemechanismus (7) einen Rahmen (16) aufweist, der mit dem zumindest einen Steuerelement (8a; 8b) versehen ist, und der an der Rückenlehne (4) mit der Möglichkeit befestigt ist, um sich in Bezug auf die Rückenlehne (4) um eine dritte Drehachse (Z) derart zu drehen, dass sich das zumindest eine Steuerelement (8a; 8b) in einen Bereich hinter dem Sitz (100) und gegenüber dem Aufnahmebereich (6) in Bezug auf die Rückenlehne (4) dreht, wenn das zumindest eine Steuerelement (8a; 8b) von der ersten Position in die zweite Position bewegt wird.

10. Sitz nach einem der vorhergehenden Ansprüche, wobei der zumindest eine Aktuator (9a) eine Gasfeder (17) aufweist, die am Sitzelement (1) und der Basis (2) angelenkt ist, um das Sitzelement (1) um die erste Drehachse (X) zu drehen, und das zumindest eine Steuerelement (8a) vorgesehen ist, um die Gasfeder (17) zu betätigen, um das Sitzelement (1) in Bezug auf eine Drehung um die erste Drehachse (X) zu bewegen, wenn sich das zumindest eine Steuerelement (8a) in der zweiten Position befindet.

11. Sitz nach einem der vorhergehenden Ansprüche, wobei der zumindest eine Aktuator (9b) eine Gasfeder (18) aufweist, die am Sitzelement (1) und der Rückenlehne (4) angelenkt ist, um die Rückenlehne (4) um die zweite Drehachse (Y) zu drehen, und das zumindest eine Steuerelement (8b) vorgesehen ist, um die Gasfeder (18) zu betätigen, um die Rückenlehne (4) in Bezug auf eine Drehung um die zweite Drehachse (Y) zu bewegen, wenn sich das zumindest eine Steuerelement (8b) in der zweiten Position befindet.

12. Sitz nach einem der vorhergehenden Ansprüche, wobei der zumindest eine Aktuator einen ersten Aktuator (9a), der mit dem Sitzelement (1) wirkverbunden ist, und einen zweiten Aktuator (9b) aufweist, der mit der Rückenlehne (4) wirkverbunden ist, und das zumindest eine Steuerelement ein erstes Steuerelement (8a) und ein zweites Steuerelement (8b) aufweist, das gegenüber dem ersten Steuerelement (8a) in Bezug auf die Rückenlehne (4) angeordnet ist, wobei das erste Steuerelement (8a) und das zweite Steuerelement (8b) mit dem ersten Aktuator (9a) bzw. dem zweiten Aktuator (9b) wirkverbunden sind, um das Sitzelement (1) bzw. die Rückenlehne (4) in Bezug auf eine Drehung um jeweilige Drehachsen zu bewegen, wenn sich das erste Steuerelement (8a) und das zweite Steuerelement (8b) jeweils in der zweiten Position befinden.

13. Sitz nach Anspruch 12, wobei der Rahmen (16) des Verstellmechanismus (7) T-förmig ist und ein zentrales Element (21) aufweist, von dem sich zwei Rahmenabschnitte (22, 23), die jeweils mit dem ersten Steuerelement (8a) und dem zweiten Steuerelement (8b) versehen sind, in entgegengesetzte Richtungen erstrecken, wobei das zentrale Element (21) des Rahmens (16) an der Rückenlehne (4) mit der Möglichkeit befestigt ist, um sich um die dritte Drehachse (Z) zu drehen.

14. Sitz nach Anspruch 12 oder 13, wobei das erste Steuerelement (8a) und das zweite Steuerelement (8b) jeweilige Betätigungsflächen (10) aufweisen, wobei jede Betätigungsfläche (10) ein erstes seitliches Ende (13), das in Bezug auf die Rückenlehne (4) proximal ist, und ein zweites seitliches Ende (14) aufweist, das in Bezug auf die Rückenlehne (4) distal ist und dem ersten seitlichen Ende gegenüberliegt (13), wobei der Abstand zwischen dem ersten seitlichen Ende (13) des ersten Steuerelements (8a) und dem ersten seitlichen Ende (13) des zweiten Steuerelements (8b) maximal 40 cm beträgt und/oder der Abstand zwischen dem zweiten seitlichen Ende (14) des ersten Steuerelements (8a) und dem zweiten seitlichen Ende (14) des zweiten Steuerelements (8b) zumindest 50 cm beträgt.

## Revendications

1. Siège (100) comprenant :
- un élément de siège (1) qui est fixé à une base (2) avec la capacité de tourner par rapport à ladite base (2) autour d'un premier axe de rotation (X),
- un dossier (4) qui est fixé audit élément de siège (1) avec la capacité de tourner par rapport audit élément de siège (1) autour d'un deuxième axe de rotation (Y), le dossier (4) étant doté d'une surface de support (5) qui s'étend depuis une arête inférieure (5a) de celle-ci à une arête supérieure (5b) de celle-ci opposée à ladite arête inférieure (5a), ladite surface de support (5) délimitant avec ledit élément de siège (1) une zone de réception (6) apte à recevoir un utilisateur dudit siège (100),
- un mécanisme d'inclinaison (7) comprenant :
- au moins un élément de commande (8a ; 8b) qui est agencé latéralement audit dossier (4), à côté de ce dernier par rapport à ladite surface de support (5), dans lequel ledit au moins un élément de commande (8a ; 8b) est mobile par rapport au dossier (4) entre une première position et une deuxième position, la première position étant plus près de ladite zone de réception (6) que la deuxième position, et
- au moins un actionneur (9a ; 9b) qui est relié en fonctionnement à un dudit élément de siège (1) et dudit dossier (4) et qui est prévu pour pousser l'un dudit élément de siège (1) et dudit dossier (4) en termes de rotation autour de l'axe de rotation (X, Y) respectif,
dans lequel ledit au moins un élément de commande (8a ; 8b) est relié en fonctionnement audit au moins un actionneur (9a ; 9b) et est prévu pour actionner ledit au moins un actionneur (9a ; 9b) afin de pousser ledit un dudit élément de siège (1) et dudit dossier (4) en termes de rotation autour de l'axe de rotation (X, Y) respectif lorsque ledit au moins un élément de commande (8a ; 8b) est dans la deuxième position, **caractérisé en ce que** ledit au moins un élément de commande (8a ; 8b) est fixé audit dossier (4).

2. Siège selon la revendication 1, dans lequel ledit au moins un élément de commande (8a ; 8b) est fixé audit dossier (4) de sorte que la rotation dudit dossier (4) autour dudit deuxième axe de rotation (Y) entraîne un déplacement, de préférence une rotation, de l'au moins un élément de commande (8a ; 8b) autour dudit deuxième axe de rotation (Y).

3. Siège selon l'une quelconque des revendications précédentes, dans lequel ledit au moins un élément de commande (8a ; 8b) comprend une surface opérationnelle (10) dirigée vers ladite zone de réception (6) lorsque ledit au moins un élément de commande (8a ; 8b) est dans ladite première position.

4. Siège selon la revendication 3, dans lequel ladite surface opérationnelle (10) est agencée dans une position au-dessus dudit élément de siège (1) par rapport à ladite base (2).

5. Siège selon la revendication 3 ou 4, dans lequel ladite surface opérationnelle (10) est dirigée vers un plan de référence (29) opposé à ladite surface de support (5) par rapport audit élément de siège (1).

6. Siège selon l'une quelconque des revendications 3 à 5, dans lequel ladite surface opérationnelle (10) comprend une portion de contact (10a) qui est espacée d'une surface supérieure (1a) dudit élément (1) d'au moins 19 cm et/ou à un maximum de 32 cm lorsque ledit au moins un élément de commande (8a, 8b) est dans la première position, et dans lequel ladite portion de contact (10a) est espacée d'un axe de référence (5e) dudit dossier (4) d'au moins 23 cm et/ou à un maximum de 32 cm lorsque ledit au moins un élément de commande (8a, 8b) est dans la première position, ledit axe de référence (5e) passant à travers l'arête inférieure (5a) et l'arête supérieure (5b) de ladite surface de support (5) centralement par rapport à ladite surface supérieure (1a).

7. Siège selon l'une quelconque des revendications 3 à 6, dans lequel ladite surface opérationnelle (10) est située derrière ladite surface de support (5) dudit dossier (4) par rapport à ladite zone de réception (6) lorsque ledit au moins un élément de commande (8a ; 8b) est dans ladite première position et/ou dans ladite deuxième position.

8. Siège selon l'une quelconque des revendications 3 à 7, dans lequel ledit siège (100) comprend un dispositif de rappel élastique (15) relié en fonctionnement audit au moins un élément de commande (8a ; 8b) et prévu pour déplacer ledit au moins un élément de commande (8 ; 8b) de ladite deuxième position à ladite première position lorsque la pression externe n'agit pas sur ladite surface opérationnelle (10).

9. Siège selon l'une quelconque des revendications précédentes, dans lequel ledit mécanisme d'inclinaison (7) comprend un cadre (16) qui est doté dudit au moins un élément de commande (8a ; 8b) et qui est fixé audit dossier (4) avec la capacité de tourner par rapport audit dossier (4) autour d'un troisième axe de rotation (Z) de telle manière que ledit au moins un élément de commande (8a ; 8b) tourne vers une zone derrière ledit siège (100) et opposée à la zone de réception (6) par rapport audit dossier (4) lorsque ledit au moins un élément de commande (8a ; 8b) est déplacé de ladite première position à ladite deuxième position.

10. Siège selon l'une quelconque des revendications précédentes, dans lequel ledit au moins un actionneur (9a) comprend un ressort à gaz (17) qui est articulé sur ledit élément de siège (1) et ladite base (2) afin de tourner ledit élément de siège (1) autour dudit premier axe de rotation (X) et ledit au moins un élément de commande (8a) est prévu pour actionner ledit ressort à gaz (17) afin de pousser ledit élément de siège (1) en termes de rotation autour dudit premier axe de rotation (X) lorsque ledit au moins un élément de commande (8a) est dans la deuxième position.

11. Siège selon l'une quelconque des revendications précédentes, dans lequel ledit au moins un actionneur (9b) comprend un ressort à gaz (18) qui est articulé sur ledit élément de siège (1) et ledit dossier (4) afin de tourner ledit dossier (4) autour dudit deuxième axe de rotation (Y) et ledit au moins un élément de commande (8b) est prévu pour actionner ledit ressort à gaz (18) afin de pousser ledit dossier (4) en termes de rotation autour dudit deuxième axe de rotation (Y) lorsque ledit au moins un élément de commande (8b) est dans la deuxième position.

12. Siège selon l'une quelconque des revendications précédentes, dans lequel ledit au moins un actionneur comprend un premier actionneur (9a) relié en fonctionnement audit élément de siège (1) et un deuxième actionneur (9b) relié en fonctionnement audit dossier (4), et ledit au moins un élément de commande comprend un premier élément de commande (8a) et un deuxième élément de commande (8b) agencé à l'opposé dudit premier élément de commande (8a) par rapport audit dossier (4), dans lequel ledit premier élément de commande (8a) et ledit deuxième élément de commande (8b) sont reliés en fonctionnement audit premier actionneur (9a) et, respectivement, ledit deuxième actionneur (9b) pour pousser ledit élément de siège (1) et ledit dossier (4) respectivement, en termes de rotation autour d'axes de rotation respectifs lorsque le premier élément de commande (8a) et ledit deuxième élément de commande (8b) respectivement sont dans la deuxième position.

13. Siège selon la revendication 12, dans lequel ledit cadre (16) dudit mécanisme d'inclinaison (7) est en forme de T et comprend un élément central (21), depuis lequel deux portions de cadre (22, 23) qui sont dotées dudit premier élément de commande (8a) et dudit deuxième élément de commande (8b) respectivement, s'étendent dans des directions opposées, ledit élément central (21) dudit cadre (16) étant fixé audit dossier (4) avec la capacité de tourner autour dudit troisième axe de rotation (Z).

14. Siège selon la revendication 12 ou 13, dans lequel ledit premier élément de commande (8a) et ledit deuxième élément de commande (8b) comprennent des surfaces opérationnelles (10) respectives, chaque surface opérationnelle (10) comprenant une première extrémité latérale (13) qui est proximale par rapport audit dossier (4) et une deuxième extrémité latérale (14) qui est distale par rapport audit dossier (4) et opposée à ladite première extrémité latérale (13), dans lequel la distance entre ladite première extrémité latérale (13) du premier élément de commande (8a) et ladite première extrémité latérale (13) du deuxième élément de commande (8b) est à un maximum de 40 cm et/ou la distance entre ladite deuxième extrémité latérale (14) du premier élément de commande (8a) et ladite deuxième extrémité latérale (14) du deuxième élément de commande (8b) est au moins de 50 cm.
